(19) **Europäisches Patentamt**

**European Patent Office**

**Office européen des brevets**

(11) Veröffentlichungsnummer: **0 251 121**
**A2**

(12) ## EUROPÄISCHE PATENTANMELDUNG

(21) Anmeldenummer: **87108953.8**

(51) Int. Cl.4 **A61K 39/385**

(22) Anmeldetag: **23.06.87**

(30) Priorität: **28.06.86 DE 3621719**

(43) Veröffentlichungstag der Anmeldung:
**07.01.88 Patentblatt 88/01**

(84) Benannte Vertragsstaaten:
**CH DE FR GB LI SE**

(71) Anmelder: **Röhm GmbH**
**Kirschenallee Postfach 4242**
**D-6100 Darmstadt 1(DE)**

(72) Erfinder: **Hartl, Roland**
**Waldstrasse 1**
**D-6115 Münster-Altheim(DE)**
Erfinder: **Krämer, Dieter, Dr.**
**An der Favorite 4**
**D-6500 Mainz(DE)**

(54) **Verfahren zur Auslösung der Bildung von Antikörpern.**

(57) Antigen wirksame Stoffe mit Amino-und/oder Carboxylgruppen werden unter Erhaltung ihres elektrochemischen Zustandes, d.h. unter Erhaltung der Amino-und Carboxylgruppen kovalent an einen im Organismus eines Vertebraten nicht abbaubaren partikelförmigen Träger gebunden und als Antigen in den Organismus eines Vertebraten eingebracht, wo eine Immunantwort von hoher Spezifität und lange anhaltender Dauer hervorgerufen wird.

EP 0 251 121 A2

Antikörper finden breite Anwendung zu diagnostischen und therapeutischen Zwecken in der Human-und Tiermedizin, sowie für analytische Zwecke im Bereich der Biochemie. Seren zur Therapie und Prophylaxe von Viruserkrankungen sind klassische Antikörperpräparate, die seit vielen Jahrzehnten gebräuchlich sind.

Die übliche Methode zur Erzeugung von Antikörpern besteht in der Verabreichung eines Antigens in den Organismus eines Vertebraten, der als sog. Immunantwort Antikörper erzeugt. Diese lassen sich entweder direkt aus dem Blut des Vertebraten gewinnen oder man entnimmt dem behandelten Organismus Antikörper erzeugende Zellen und entwickelt diese zu Antikörper produzierenden Zellkulturen weiter.

Da die Antigene im Organismus des Vertebraten in der Regel rasch abgebaut werden, muß das Antigen meistens mehrfach verabreicht werden, bis es zu einer hinreichend starken Antikörperbildung kommt.

Man hat schon Antigene an partikelförmige Trägersubstanzen gebunden, die selbst nicht im Organismus des Vertebraten abgebaut werden. Solche trägergebundenen Antigene vermögen in manchen Fällen eine verstärkte oder verlängerte Immunantwort auszulösen.

Die Bindung des Antigens an den Träger kann durch Adsorption erfolgen. Beispielsweise wurden Viren, Fab-Fragmente oder Pollenantigene adsorptiv an Aluminiumhydroxid-Gele, mikrokristalline Cellulose, synthetische Polymere auf Basis von Acrylamid, Methylmethacrylat oder ionischen Vinylmonomeren gebunden (vgl. US 3 651 213; G.T. Stevenson, Nature 1974, 247 (5441), S. 477-478; DE-OS 19 42 196; DE-OS 19 42 161). Die adsorptive Bindung hat den Vorteil, daß das Antigen durch den Bindungsvorgang nicht verändert wird, aber den Nachteil, daß die Bindung reversibel ist, so daß das Antigen zwar langsamer als im ungebundenen Zustand, aber trotzdem unaufhaltsam verloren geht.

U. Gröschel-Stewart et.al. hatten Antigen-Proteine kovalent an Polyacrylamid-Träger gebunden (U. Gröschel-Stewart et al. Histochemistry 50, 271-279 (1977)) und damit eine Immunantwort im Kaninchen-Organismus erhalten. Die kovalente Bindung greift Aminogruppen des Antigens unter Bildung von Amidgruppen an. Im Gegensatz zu der ursprünglichen Aminogruppe ist die Amidgruppe elektrisch neutral. Der elektrochemische Zustand des Antigens wird also durch diese Bindung über Amidgruppen verändert. Das hat zur Folge, daß auch die Immunantwort verändert und weniger spezifisch für das nicht gebundene Antigen ist. Weiterhin ist die Amidbindung nicht hydrolysebeständig.

Der Erfindung liegt die Aufgabe zugrunde, bei der Einbringung des Antigens in den Organismus eines Vertebraten die Bildung von Antikörpern von erhöhter Spezifität auszulösen und weiterhin die Dauer der Antikörperbildung möglichst zu verlängern.

Die Aufgabe wird erfindungsgemäß durch das in den Patentansprüchen angegebene Verfahren gelöst. Die Erfindung bedient sich solcher an sich bekannter Bindungsverfahren, bei denen die Carboxyl-und Aminogruppen des antigen wirksamen Stoffes nicht so in den Bindungsvorgang einbezogen werden, daß ihr elektrochemischer Zustand dabei verändert wird. Der elektrochemische Zustand wird dadurch bedingt, daß Carboxyl-und Aminogruppen in Abhängigkeit vom pH-Wert oder ihrer Wechselwirkung mit geladenen Teilchen in elektrisch geladene Ionen, also Carboxylat-bzw. Ammoniumionen, übergehen können. Diese Fähigkeit bleibt bei der Bindung gemäß der vorliegenden Erfindung erhalten.

Infolge des unveränderten elektrochemischen Zustands des gebunden antigen wirksamen Stoffes wird eine Immunantwort im Organismus des Vertebraten hervorgerufen, die eine höhere Spezifität gegenüber dem ungebundenen antigen wirksamen Stoff aufweist, als wenn die Immunantwort mit einem unter Veränderung des elektrochemischen Zustands gebundenen antigen wirksamen Stoff ausgelöst worden wäre.

Zur kovalenten Bindung antigen wirksamer Stoffe, die Amino-und/oder Carboxylgruppen aufweisen, an ein im Organismus des Vertebraten nicht abbaubares partikelförmiges Trägermaterial bestehen verschiedene Möglichkeiten, wobei der Einsatz eines in bestimmter Weise aufgebauten Trägermaterials von ausschlaggebender Bedeutung ist.

Bevorzugt sind Trägermaterialien auf Basis synthetischer Polymerer, die Oxirangruppen enthalten. Die Oxirangruppen vermögen unter milden Bedingungen mit den primären oder sekundären Aminogruppen kovalent zu einer beta-Hydroxyäthylgruppe, die als zusätzlicher Substituent an die Aminogruppe tritt, zu reagieren, wobei primäre in sekundäre und sekundäre in tertiäre Aminogruppen übergehen. Der elektrochemische Zustand bleibt dabei praktisch unverändert. Aus EP-B 54 249 sind Teilchen mit einem mittleren Durchmesser von 0,03 bis 10 μm bekannt, die aus einem vernetzten Polymerisat von Glycidyl(meth)-acrylat bzw. dessen Mischpolymerisat mit hydrophilen Comonomeren bestehen, an welches immunogen wirkende Stoffe, wie Antigene oder Antikörper, kovalent gebunden sind. Der Einsatz derartiger Immunoteilchen zur Auslösung der Antikörperbildung im Organismus eines Vertebraten ist nicht bekannt. Größere Trägerteilchen zur Bindung von biologisch aktiven Wirkstoffen werden gemäß DE-C 27 22 751 aus Oxirangruppen enthaltenden Monomeren, Acrylamid bzw. Methacrylamid und/oder Methylenbis-(meth)acrylamid hergestellt. Sie haben Durchmesser von 5 bis 1000 μm.

Eine weitere Bindungsmethode beruht auf dem in DE-C 22 63 289 beschriebenen Verfahren. Sie eignet sich besonders für antigen wirksame Stoffe mit Peptidstruktur, wobei insbesondere Einheiten des Glycins, Cysteins, Cystins, Methionins oder Tryptophans bindungsfähig sind. Die Bindungsreaktion erfolgt nach einem radikalischen Mechanismus zwischen einer Kohlenwasserstoffgruppe des Peptids und einer radikalisch aktivierbaren Gruppe des Trägerpolymeren, wobei die Amino- und Carboxylgruppen unverändert bleiben. Radikalisch aktivierbare Gruppen in diesem Sinne sind beispielsweise Alkenyl-und Alkylphenylgruppen, vorzugsweise Allyl-und Toluylgruppen. Die Aktivierung erfolgt in Anwesenheit eines Photosensibilisators mittels ultraviolettem Licht oder - bei zusätzlicher Anwesenheit eines organischen Peroxids - mittels sichtbarem Licht in Abwesenheit von Sauerstoff.

Die Bindung antigen wirksamer Stoffe mit Peptidstruktur an ein synthetisches Trägerpolymermaterial, das olefinische C = C-Doppelbindungen enthält, gelingt nach dem in der DE-C 24 30 128 beschriebenen Verfahren in Gegenwart einer zu Radikalen zerfallenden Verbindung oder eines Redoxsystems. Im Gegensatz zu Einschlußverfahren, bei denen antigen wirksame Stoffe ohne kovalente Bindung in die Matrix eines entstehenden Vinylpolymeren eingebunden wird, wird das hier besprochene Verfahren in Abwesenheit radikalisch polymerisierbarer Monomerer durchgeführt.

Die Bindung von Peptiden oder anderen biogenen Stoffen mit antigener Wirkung an latexförmige Träger ist in der EP-A 65 069 beschrieben. Sofern dort beschriebene Methoden für die Zwecke der vorliegenden Erfindung angewendet werden, müssen solche bindungsaktive Gruppen ausgewählt werden, die eine kovalente Bindung unter Erhaltung der Amino-und Carboxylgruppen des antigen wirksamen Stoffes erzeugen. Ungeeignet sind also aktivierte Carbon-oder Sulfonsäuregruppen, welche mit Aminogruppen unter Amidbildung reagieren würden. Außer den schon erwähnten Oxirangruppen sind zur Bindung z.B. Halogenalkyl-und Halogen-alkoyl-Gruppen geeignet.

Eine wesentliche Eigenschaft der Trägerpartikel ist ihre Beständigkeit gegen Abbau im Organismus des Vertebraten. Als nicht abbaubar im Sinne der Erfindung sind Träger anzusehen, die wenigstens solange ihre Trägerfunktion ausüben, wie der daran gebundene Stoff seine antigene Wirksamkeit beibehält. Diese Eigenschaft besitzen vor allem synthetische Vinylpolymere, da sie eine durchlaufende Kohlenstoffkette besitzen, die durch die in Organismen weitverbreiteten hydrolytischen Enzyme nicht spaltbar ist.

Die bereits erwähnten Patentschriften beschreiben geeignete Trägerpolymere im Detail. Charakteristische Aufbaukomponenten dieser Polymeren sind:

1. Monomere mit bindungsaktiven Gruppen, wie

a) Oxirangruppen enthaltende Monomere, die mit Aminogruppen des antigen wirksamen Stoffes unter Öffnung des Oxiranringes und Alkylierung des Stickstoffatoms reagieren. Beispiele sind: Glycidylacrylat und -methacrylat, Alkylglycidyläther;

b) Halogenalkylgruppen enthaltende Monomere, insbesondere Chloralkyl-oder Bromalkylverbindungen, die mit Aminogruppen unter N-Alkylierung reagieren. Beispiele sind: Chloracetoxyalkyl(meth)acrylate, Bromalkyl(meth)acrylate, Chloressigsäurevinylester;

c) Aromatische Gruppen enthaltende Monomere, die unter Einwirkung einer aktivierenden Strahlung mit einer Kohlenwasserstoffgruppierung des antigen wirksamen Stoffes unter Ausbildung einer kovalenten C-C-Bindung reagieren. Dazu gehören: Styrol, Vinyltoluol, Benzylacrylat und -methacrylat;

d) Alkenyl-Seitengruppen neben einer radikalisch polymerisierbaren Vinyl-bzw. Vinylidengruppe enthaltende Monomere, die bei Anregung durch eine aktivierende Strahlung oder durch chemisch erzeugte Radikale mit einer Kohlenwasserstoffgruppierung des antigen wirksamen Stoffes unter Ausbildung einer kovalenten C-C-Bindung zu reagieren vermögen. Beispiele für geeignete Monomere sind: Allylacrylat und -methacrylat, Polyallylester mehrbasischer Säuren wie Triallylcyanurat.

2. Vernetzende Monomere mit 2 oder mehr radikalisch polymerisierbaren Kohlenstoffdoppelbindungen im Molekül. Die in 1 d erwähnten Monomeren wirken teilweise bei der Polymerisation vernetzend. Im Gegensatz zu den dort genannten Monomeren mit Alkenylgruppen enthalten vernetzende Monomere wenigstens zwei Kohlenstoffdoppelbindungen,die durch benachbarte elektronenanziehende Gruppen aktiviert sind. Beispiele sind Diacrylate oder Dimethacrylate von Glykolen, wie Äthylenglykol, Propylenglykol oder Butylenglykol, Tri-und Tetraacrylate bzw. Tri-und Tetramethacrylate von 3-oder 4-wertigen Alkoholen, wie Trimethylolpropan oder Pentaerythrit; Methylen-bis-acrylamid oder -methacrylamid, Divinylbenzol.

3. Hydrophile Monomere, die sich durch eine gewisse Wasserlöslichkeit auszeichnen. Vorzugsweise bilden sie wenigstens 10 %ige wäßrige Lösungen bei 25 Grad Celsius. Dazu gehören Acrylamid, Methacrylamid, Vinylpyrrolidon, Hydroxyalkylester der Acryl-oder Methacrylsäure, vorzugsweise mit 2 bis 4 C-Atomen im Hydroxyalkylrest. Diese Monomeren sind bevorzugt, weil sie elektroneutral sind und den elektrochemischen Zustand des gebunden antigen wirksamen Stoffes nicht beeinflussen. In manchen Fällen können auch ionisierbare Monomere verwendet werden; dazu gehören äthylenisch ungesättigte, polymerisierbare Mono-und Dicarbonsäuren sowie ihre wasserlöslichen Salze, beispielsweise Acryl-, Methacryl-, Itakon-, Malein-oder Fumarsäure. Weiterhin gehören Aminoalkylester und Aminoalkylamide von ungesättigten Carbonsäuren, wie Dialkylaminoalkylester oder Dialkylaminoalkylamide der Acryl-und Methacrylsäure, die vorzugsweise 1 bis 5 C-Atome in den Alkylresten enthalten, zu dieser Gruppe.

Hohe Anteile an hydrophilen Monomeren machen das Polymer wasserlöslich, was für den Einsatz als partikelförmiges Antigen ungeeignet ist. Daher werden in diesem Falle ausreichende Mengen an vernetzenden Monomeren mitverwendet, um das Polymer wasserunlöslich zu machen. Niedrige Vernetzeranteile, z.B. unter 5 Gew.-%, ergeben im allgemeinen gelartig quellbare Polymere, während hohe Vernetzeranteile nicht quellbare makroporöse Teilchen oder solche mit Hohlperlstruktur ergeben.

4. Hydrophobe Monomere, die sich durch eine Wasserlöslichkeit unter 10 % bei 25 Grad Celsius auszeichnen. Sie können beispielsweise angewendet werden, wenn das Trägerpolymer in Latexform hergestellt wird. In manchen Fällen vermögen sie durch hydrophobe Wechselwirkung mit dem antigen wirksamen Stoff dessen Bindung zu fördern. Die unter 1c und 1d genannten Monomeren fallen gleichzeitig in die Gruppe der hydrophoben Monomeren. Weiterhin gehören dazu die Ester von polymerisierbaren ungesättigten Mono-und Dicarbonsäuren mit aliphatischen Alkoholen, die vorzugsweise 1 bis 12, insbesondere 1 bis 4 C-Atome enthalten, beispielsweise Methyl-, Äthyl-oder Butyl-acrylat oder -methacrylat, ferner Olefine wie Äthylen oder Propylen, Vinylchlorid, Vinylester gesättigter aliphatischer Carbonsäuren mit 2 bis 10 C-Atomen.

Die relativen Anteile der beteiligten Monomeren richten sich nach ihrer Funktion, d.h. dem für ihre Wirkung erforderlichen Bedarf. Soweit nicht in den oben erwähnten Patentschriften bereits geeignete Monomeranteile angegeben sind, können folgende Bereiche als Richtwerte dienen.

1) Bindungsaktive Monomere: 1 bis 80 Gew.-% vorzugsweise 5 bis 50 Gew.-%

2) Vernetzende Monomere: 0 bis 50 Gew.-%, vorzugsweise 0,01 bis 30 Gew.-%

3) Hydrophile Monomere 0 bis 99 Gew.-%, vorzugsweise 20 bis 95 Gew.-%

4) Hydrophobe Monomere 0 bis 50 Gew.-%, vorzugsweise 0 bis 30 Gew.-%.

Für die angestrebte Wirkung der Trägerpolymeren ist es ausreichend, wenn die äußerste Schicht der Polymerteilchen, bei porösen bzw. makroporösen Polymerteilchen auch die Porenwandungen, aus dem beschriebenen Polymermaterial bestehen. Darunter kann sich ein Kern aus einem anderen Material befinden, beispielsweise ein anorganischer Trägerkörper oder ein Saatlatexkern aus einem hydrophoben Polymer, wie Polystyrol, Poly(meth)acrylestern u. dergl.

Die Größe der Teilchen des Trägerpolymeren richtet sich nach der Art der Einbringung in den Organismus des Vertebraten. Wird das teilchenförmige Antigen in die Blutbahn eingebracht, so liegt die Teilchengröße vorzugsweise im Bereich von 0,01 bis 10 µm. Bei Einlagerung in das Gewebe können auch größere Teilchen, beispielsweise bis zu 100 µm verwendet werden. Trägerteilchen im Bereich von 0,01 bis 2 µm werden vorteilhaft durch Emulsionspolymerisation erzeugt. Teilchen in der Größenordnung von 1 bis 50 µm sind durch Fällungspolymerisation in einem Medium, welches ein Lösungsmittel für die Monomeren und ein Nichtlösungsmittel für die Polymeren darstellt, zugänglich. Die Suspensions-bzw. Perlpolymerisation in einem Medium, das weder die Monomerenphase noch die Polymerteilchen löst, führt zu Teilchen zwischen 2 und 100 µm. Gröbere Teilchen oder Substanzpolymerisate können durch Schlagmühlen, Homogenisatoren u.dergl. zu Teilchen in diesen Bereichen zerkleinert werden. Das Mengenverhältnis von antigen wirksamem Stoff und Trägermaterial kann in einem weiten Bereich liegen. Eine hohe Wirksamkeit wird im allgemeinen im Bereich zwischen 1 : 10 und 1 : 1000 erreicht.

Unter antigen wirksamen Stoffen werden alle Stoffe verstanden, die im Organismus eines Vertebraten eine Immunreaktion auszulösen vermögen. Dazu gehören praktisch alle körperfremden, natürlichen oder synthetischen Makromoleküle mit Molekulargewichten über 2000, ebenso Oberflächenstrukturen von Fremdpartikeln, wie aktive oder inaktivierte Bakterien oder Viren, die aus derartigen Makromolekülen aufgebaut sind. Die Erfindung bezweckt den Einsatz von solchen antigen wirksamen Stoffen, die eine oder mehrere Carboxyl-und/oder Aminogruppen enthalten. Das Verfahren ist mit antigen wirksamen Stoffen ohne Carboxyl-und Aminogruppen in gleicher Weise durchführbar, jedoch entfällt dann der erfindungsgemäße Vorteil, der auf der Erhaltung des elektrochemischen Zustands beruht. Den Amino-bzw. Carboxylgruppen stehen die durch Salzbildung daraus entstehenden Ammonium-bzw. Carboxylatgruppen gleich. Die Aminogruppen können primär, sekundär oder tertiär sein. Obwohl die Anwesenheit von jeweils wenigstens einer Aminogruppe und Carboxylgruppe die Regel ist, gehören antigen wirksame Stoffe, die lediglich eine oder mehrere Aminogruppen aber keine Carboxylgruppe, oder eine oder mehrere Carboxylgruppen, aber keine Aminogruppe enthalten, zum Umfang der Erfindung.

Zu den antigen wirksamen Stoffen im Sinne der Erfindung gehören auch Haptene; das sind Stoffe, die im ungebundenen Zustand allein kein Antigen darstellen, sondern erst nach der Bindung an einen Träger zum Antigen werden. In der Praxis ist die Frage, ob ein bestimmter Stoff auch ungebunden als Antigen wirkt, schwer zu entscheiden, weil die Immunantwort in manchen Fällen erst nach einer Bindung des Stoffes an eine ungelöste körpereigene Substanz ausgelöst wird. Es gibt daher keine Grenze grundsätzlicher Art für das Molekulargewicht oder die Größe des antigen wirksamen Stoffes. Der Stoff kann in Wasser löslich oder unlöslich sein und muß im letztgenannten Fall in einer für die kovalente Bindung geeigneten Flüssigkeit löslich sein.

Eine herausragende Gruppe von antigen wirksamen Stoffen sind solche mit Protein-bzw. Peptidstruktur, die allein oder in Verbindung mit nicht protein-bzw. peptidartigen Anteilen vorkommen kann. Peptide und Proteine enthalten in der Regel je eine Amino-und eine Carboxylgruppe als Endgruppen. Weitere Gruppen von praktischer Bedeutung sind Polysaccharide und Nukleinsäuren. Soweit sie nicht selbst Carboxyl-oder Aminogruppen tragen, können sie mit solche Gruppen enthaltenden Stoffen verbunden sein. Unter den Haptenen seien Penicilline, Tetracyline und Digoxin als Beispiele genannt.

Die Vertebraten, die erfindungsgemäß behandelt werden, sind vorzugsweise Warmblüter. Geeignet sind z.B. Rinder, Schafe, Ziegen, Pferde, Schweine, Hunde, Katzen, Kaninchen, Ratten und Mäuse unter den Säugetieren und Hühner, Enten, Gänse, Puten, Tauben unter den Vögeln. Die Erfindung ist auch auf Kaltblüter anwendbar, wie Fische und Reptilien.

Zur Applikation kann das partikelförmige Antigen in einer isotonischen Lösung .suspendiert und in die Blutbahn gespritzt oder in das Haut-oder Bindegewebe eingelagert werden. Die Immunantwort kann meistens nach einer Inkubationszeit von 3 bis 20 Tagen beobachtet werden. In der Regel genügt dafür die einmalige Applikation des Antigens. Die Entnahme von Antikörper enthaltendem Blut bzw. Serum oder von Antikörper erzeugenden Zellen für die Anlage von Zellkulturen erfolgt in an sich bekannter Weise wie nach der Auslösung der Immunantwort mit nicht an Trägerpartikel gebundenem Antigen.

Beispiel:

2 g eines pulverförmigen, zu Teilchen von etwa 1 Mikrometer Durchmesser redispergierbaren Trägermaterials (Handelsprodukt EUPERGIT CIZ, Röhm GmbH, Darmstadt), das bindungsaktive Oxiran-Gruppen enthält (hergestellt gemäß DE 31 16 995; bzw. US Patent Appl. Serial No. 930 023) werden in 4 ml einer Pufferlösung vom pH 7,3 (I,0 M Na-Phosphat + 0,I5 NaCl) suspendiert und 20 mg Rinderserumalbumin ("BSA", Hersteller Miles Lab, Lot No. 63, Code No.8I-00I), zugesetzt. Man läßt die Suspension 72 Stunden bei Raumtemperatur stehen. Danach wird mehrmals mit Wasser gewaschen und zentrifugiert. Der Proteintest nach Bradford (ausgeführt mittels Testkit der Fa. Bio-Rad) ergibt einen Gehalt von 4,4 mg kovalent gebundenem BSA je Gramm Trägermaterial.

455 mg des erhaltenen Immobilisats, enthaltend 2,0 mg BSA werden mit I ml inkomplettem Freund'schem Adjuvans (einem in der Immunchemie allg. bekannten Hilfsmittel) vermischt und 40 min mittels Ultraschall zu einer Wasser-in-Öl-Emulsion suspendiert. Die Suspension wird auf I0 Einzeldosen verteilt einem Kaninchen subcutan appliziert. Die ganze Behandlung wird nach drei Wochen zur Verstärkung des Immuneffekts wiederholt. Nach weiteren vier Wochen werden dem behandelten Kaninchen I0 ml Blut aus der Ohrvene entnommen. Das abgenommene Blut wird eine Stunde bei 37°C und weitere 36 Stunden im Kühlschrank stehengelassen. Dann wird in üblicher Weise das Anti-Serum vom Blutkuchen abgetrennt.

Die Antikörper-Konzentration in dem Antiserum läßt sich auf einfache Weise mit dem Kapillartest messen. Hierzu wird das Antigen, nämlich BSA, zu einer Konzentration von I mg/ml in PBS (phospate buffer, saline, pH 7,3) gelöst. Die Lösung wird in einer Füllhöhe von 3 cm in eine Glaskapillare eingefüllt. Dann zieht man durch Schräghalten der Kapillare etwa die gleiche Menge des aus dem Kaninchenblut gewonnenen Antiserums in dieselbe Kapillare ein und verschließt das untere Ende der Kapillare mit Kitt. Man läßt die Kapillare I Stunde bei 37°C und weitere 48 Stunden bei 5-8°C stehen und mißt die Höhe des abgesetzten Präzipitats am unteren Ende der Kapillare. Es wird eine Präzipitathöhe von 12 mm gemessen. Eine Präzipitathöhe von I0 mm entspricht einer Antikörper-Konzentration von etwa I mg/ml. Daraus ergibt sich eine Antikörper-Konzentration von I,2 mg/ml in dem Antiserum.

Zum Vergleich wird das Blutserum eines nicht behandelten Kaninchens in der gleichen Weise mit dem Antiserum behandelt. Dabei bildet sich überhaupt kein Präzipitat in der Kapillare.

In einem weiteren Versuch wird nur das immobilisierte BSA (ohne inkomplettes Freund'sches Adjuvans) einem Kaninchen subcutan appliziert und die Behandlung wie oben beschrieben wiederholt. Beim Immuntest ergibt sich nur eine Präzipitathöhe von 4 mm was einer Antikörper-Konzentration von 0,4 mg/ml entspricht. Daraus ergibt sich die erhöhte Wirksamkeit des immobilisierten BSA in Gegenwart von imkomplettem Freund'schem Adjuvans.

In entsprechender Weise lassen sich bei Einsatz von Insulin oder Thyroid-stimulierendem Hormon ("TSH") anstelle von BSA die entsprechenden Antiseren herstellen. Sie lassen sich für diagnostische Zwecke zur Bestimmung von Insulin bzw. TSH einsetzen.

**Ansprüche**

1. Verfahren zur Auslöung der Antikörperbildung im Organismus eines Vertebraten, wobei man einen antigen wirksamen, Amino- und/oder Carboxylgruppen enthaltenden, löslichen Stoff durch kovalente Bindung an einen im Organismus des Vertebraten nicht abbaubaren partikelförmigen Träger unter Bildung eines partikelförmigen Antigens immobilisiert und das Antigen in den Organismus des Vertebraten einbringt,

dadurch gekennzeichnet,

daß man den antigen wirksamen Stoff unter Erhaltung seiner Amino-und Carboxylgruppen kovalent an den Träger bindet.

2. Verfahren nach Anspruch 1, dadurch gekennzeichnet, daß man einen Oxirangruppen enthaltenden Träger einsetzt und den antigen wirksamen Stoff durch Umsetzung mit den Oxirangruppen immobilisiert.

3. Verfahren nach Anspruch 1, dadurch gekennzeichnet, daß man einen Alkenylgruppen und/oder Alkylphenylgruppen enthaltenden Träger einsetzt und den antigen wirksamen Stoff in Anwesenheit eines Photosensibilisators mittels ultraviolettem Licht oder - bei zusätzlicher Anwesenheit eines organischen Peroxids - mittels sichtbarem Licht in Abwesenheit von Sauerstoff kovalent an dem Träger immobilisiert.

4. Verfahren nach Anspruch 1, dadurch gekennzeichnet, daß man einen Träger mit olefinischen C = C-Doppelbindungen einsetzt und den antigen wirksamen Stoff in Gegenwart einer zu Radikalen zerfallenden Verbindung oder eines Redoxsystems in Abwesenheit radikalisch polymerisierbarer Monomerer kovalent an den Träger bindet.

5. Verfahren nach einem der Ansprüche 1 bis 4, dadurch gekennzeichnet, daß man einen Träger einsetzt der zu

1) 1 - 88 Gew.-% aus bindungsaktiven Monomeren, die eine Oxirangruppe, eine Halogenalkylgruppe, eine aromatische Gruppe oder - neben einer polymerisierbaren Vinyl-oder Vinylidengruppe - eine Alkenylseitengruppe enthalten,

2) 0 - 50 Gew.-% aus vernetzenden Monomeren mit zwei oder mehr radikalisch polymerisierbaren Kohlenstoffdoppelbindungen im Molekül,

3) 0 - 99 Gew.-% aus hydrophilen Monomeren, die bei 25 Grad Celsius wenigstens 10 %ige wäßrige Lösungen bilden,

4) 0 - 50 Gew.-% aus hydrophoben Monomeren, die bei 25 Grad Celsius weniger als 10 %ige wäßrige Lösungen bilden

aufgebaut ist.

6. Verfahren nach den Ansprüchen 1 bis 5, dadurch gekennzeichnet, daß der antigen wirksame Stoff an einen partikelförmigen Träger mit einem mittleren Teilchendurchmesser zwischen 0,01 und $100\mu m$ gebunden wird.

7. Verfahren nach Anspruch 6, dadurch gekennzeichnet, daß der antigen wirksame Stoff an einen als wäßriger Latex vorliegenden Träger mit einer Teilchengröße zwischen 0,01 und $2\mu m$ gebunden wird.

8. Verfahren nach den Ansprüchen 1 bis 7, dadurch gekennzeichnet, daß als antigen wirksamer Stoff ein Stoff mit Protein-bzw. Peptidstruktur eingesetzt wird.

9. Verfahren nach den Ansprüchen 1 bis 7, dadurch gekennzeichnet, daß als antigen wirkender Stoff ein Hapten, welches eine oder mehrere Amino-und/oder Carboxylgruppen enthält, eingesetzt wird.

10. Verfahren nach den Ansprüchen 1 bis 9, dadurch gekennzeichnet, daß das partikelförmige Antigen in den Organismus eines warmblütigen Vertebraten eingebracht wird.

II. Verfahren nach Anspruch I0, dadurch gekennzeichnet, daß das partikelförmige Antigen subcutan in den Organismus des Vertebraten eingebracht wird.

12. Verfahren nach Anspruch I0, dadurch gekennzeichnet, daß das partikelförmige Antigen in Form einer Wasser-in-Öl-Emulsion in den Organismus des Vertebraten eingebracht wird.